# EUROPEAN PATENT SPECIFICATION

(11) **EP 3 842 799 B1**
(45) Date of publication and mention of the grant of the patent: **02.11.2022**
(21) Application number: 19220021.0
(22) Date of filing: 30.12.2019
(51) Int. Cl.: G01N 33/12

(54) **NON-INVASIVE METHOD TO EVALUATE THE MAIN BIOHYDROGENATION PATHWAYS OF LIPIDS IN THE RUMEN OF LAMB**
NICHTINVASIVES VERFAHREN ZUR BEWERTUNG DER HAUPTBIOHYDRIERUNGSWEGE VON LIPIDEN IM PANSEN VON LÄMMERN
PROCÉDÉ NON INVASIF POUR ÉVALUER LES PRINCIPALES VOIES DE BIOHYDROGÉNATION DES LIPIDES DANS LE RUMEN DES AGNEAUX

(30) Priority: 26.12.2019 PT 2019116022
(43) Date of publication of application: 30.06.2021
(73) Proprietor: Cebal - Centro De Biotecnologia Agrícola E Agro-Alimentar Do Alentejo, 7801-908 Beja (PT); Instituto Nacional de Investigação Agrária E Veterinária, I.P. (INIAV), 2780-157 Oieras (PT); Faculdade de Medicina Veterinária-Universidade de Lisboa, 1300-477 Lisboa (PT)
(72) Inventor: Amador de Oliveira Francisco, Alexandra Eduarda, 2070-639 Vila Chã de Ourique (PT); Almeida Alves, Susana Paula, 1400-054 Lisboa (PT); Garcia Correia Vaz Portugal, Ana Paula, 2005-099 Azóia de Baixo (PT); Sousa Jerónimo Alves, Eliana Alexandra, 7670-354 Ourique (PT); Bento Santos-Silva, José Manuel, 2005-160 Santarém (PT); Branquinho Bessa, Rui José, 2785-760 S. Domingos de Rana (PT)
(74) Representative: Ferreira, Maria Silvina

(56) References cited:
- FRANCISCO ALEXANDRA E ET AL: "Effects of alfalfa particle size and starch content in diets on feeding behaviour, intake, rumen parameters, animal performance and meat quality of growing lambs", MEAT SCIENCE, vol. 161, 107964, 15 October 2019 (2019-10-15), pages 1-15, XP085984604, ISSN: 0309-1740, DOI: 10.1016/J.MEATSCI.2019.107964 [retrieved on 2019-10-15]
- LAURA M. NEUMANN ET AL: "An investigation of the relationship between fecal and rumen bacterial concentrations in sheep", ZOO BIOLOGY, vol. 27, no. 2, 1 January 2008 (2008-01-01), pages 100-108, XP055724419, US ISSN: 0733-3188, DOI: 10.1002/zoo.20166
- HAMID MOHAMMADZADEH ET AL: "Molecular comparative assessment of the microbial ecosystem in rumen and faeces of goats fed alfalfa hay alone or combined with oats", ANAEROBE, vol. 29, 11 December 2013 (2013-12-11), pages 52-58, XP055724490, GB ISSN: 1075-9964, DOI: 10.1016/j.anaerobe.2013.11.012

## Description

### Technical field

This application relates to a non-invasive method to evaluate the biohydrogenation activity in rumen in growing lambs during fattening based on fatty acid profile of faeces evaluated by GC.

### Background art

Fatty acid (FA) composition of ruminant edible products is strongly affected by the extensive isomerization and hydrogenation of the unsaturated dietary FA mediated by rumen microbiota.

These biochemical processes, globally designated biohydrogenation (BH), confer specificity to ruminant meat and milk fat, which have a high proportion of saturated FA and large number of FA biohydrogenation isomers (BI), many with double bonds of *trans* geometry.

The main octadecenoic BI in ruminal fluid is 18:1 *trans-*11 (vaccenic acid). The main decadienoic BI is 18:2 *cis-*9,*trans*-11(rumenic acid), which may be synthesized in rumen or in tissues, by desaturation of 18:1 trans-11 mediated by the endogenous enzyme, Δ9 desaturase. However, when ruminants are fed with diets rich in soluble carbohydrates, as starch, frequently the BH pathway shift to an alternative biochemical process, resulting in a predominance of 18:1 *trans-10,* which becomes the main octadecenoic BI in ruminal fluid. In such conditions, the synthesis of rumenic acid in ruminal fluid and in tissues is very low. The ratio between 18:1 *trans-10* / 18:1 trans-11 isomers may be used as indicator of rumen BH and values higher than 1 indicate a shift to *trans*10 BH pathway (shift-*trans*10)[2]*.* The occurrence of shift-*trans*10 in lambs during fattening indoors in Mediterranean region is frequent as reported by [1]. Francisco et al., "Effects of alfalfa particle size and starch content in diets on feeding behaviour, intake, rumen parameters, animal performance and meat quality of growing lambs", Meat Science, vol. 161, 2019, pages 1-15, discloses a method for evaluating biohydrogenation pathways of lipids in the rumen of lambs by measuring the fatty acid profile in the lambs' rumen fluids.

Despite the recommendations to reduce the consumption of *trans* FA, the effect of FA in consumer's health is isomer dependent and vaccenic and rumenic acids are considered as healthy FA with potential protective effect in several important diseases of the cardiovascular or oncologic forums[4]. Oppositely 18:1 trans-10 is considered as potentially harmful for human health [5].

Several nutritional strategies have been tested to promote the synthesis and deposition in ruminant tissues of healthy FA, namely the use of forage based diets and the supplementation with polyunsaturated FA [3]. The efficacy of such nutritional strategies varies widely and there is a high individual variability in animal response [2]. The efficacy of such dietary manipulations can only be verified after the slaughter of the lambs. During fattening the FA composition of rumen fluid can be determined by direct or indirect procedures that normally are not applied in the farm. The most accurate method to evaluate ruminal BH is by direct sampling of ruminal fluid for FA analysis. To collect ruminal content in live animals two procedures may be used: a stomach tube or rumenocentesis. The first method involves the oro-ruminal passage of a tube to collect rumen fluid. The animals must be immobilized and a specialized stomach tube should be used to minimize saliva contamination. The operation must be conducted by an experimented technician, assuring that the tube reach the ventral sac of the rumen. The second method involves a percutaneous aspiration of rumen contents from the ventral ruminal sac, using a needle attached to a 10- to 20- ml syringe. Must be done by a veterinary and demands for immobilization of the animals and the use of local anesthesia. In spite of being a simple procedure, some problems may occur, such as subcutaneous or intra-abdominal abscesses, localized peritonitis, hematomas, blood contamination of the sample, etc. Both methods involve discomfort to animals, always cause some stress and/or pain, demand for trained staff and medical assistance and may not be easily applied in light lambs. Moreover, a sample of ruminal content collected in a specific moment of the day may not be representative of the overall rumen status over a 24 hours period. An indirect approach to evaluate BH may be using plasma FA composition. This method causes a variable level of stress to animals, requires medical assistance, and is less accurate than the direct methods.

### Summary

The present patent application describes a non-invasive method to monitoring the metabolization of polyunsaturated fatty acids (PUFA) in the rumen, which will directly affect the lipid composition of ruminant derived foods.

Thus, it is disclosed herein a method for evaluating the main biohydrogenation pathways of lipids in the rumen of lambs, wherein the method comprises the following steps:
a) Collection of lamb faeces;
b) Drying the lamb faeces;
c) Preparation and extraction of the fatty acid methyl ester from the dried lamb faeces;
d) Analysis of fatty acid methyl esters by gas-chromatography with flame ionization detection equipped with a highly polar capillary column of cyanopropylsiloxane as stationary phase;
e) Identification of *cis* and *trans* octadecenoic isomers (18:1);
f) application of linear regression equations (Y=aX+b) to the 18:1 trans-10, 18:1 trans-11 proportions and the ratio 18:1 trans-10/ 18:1 trans-11 fatty acids of faeces as marker of 18:1 trans-10, 18:1 trans-11 proportions and the ratio of 18:1 trans-10/ 18:1 trans-11 fatty acids in rumen contents, wherein a predominance of
the octadecenoic biohydrogenation isomers 18:1 trans-10 indicates a shift in rumen from normal 18:1 trans-11 biohydrogenation pathway to trans-10 shifted pathway. In one embodiment, the Drying step is performed by freeze-drying the lamb faeces.

According to the present invention, the ratio of 18:1 trans-10/ 18:1 trans-11 fatty acids in faeces content is used in the prediction of fatty acid composition of lamb-derived food, preferably in lamb meat.

### Disclosure/Detailed Description

The present patent application relates to a non-invasive method of obtaining information about the proportions of 18:1 trans-10, 18:1 trans-11 and the ratio 18:1 trans-10/ 18:1 trans-11 FA composition of rumen contents by analysing the FA composition of faeces. The proportions of those FA as well the ratio 18:1 trans-10 /18:1 trans-11 in faeces are highly correlated to the correspondent proportions in rumen fluid, allowing the evaluation of the main drive of BH during lambs fattening, anticipating the proportion of those BI isomers and ratio in meat. The method described herein is non-invasive, thus causing reduced or no discomfort to the lambs and that is very accurate to estimate the ruminal function, because the samples are not reported to an instantaneous moment.

### Faeces collection

The biological material can be easily collected from barn floor or directly from the lamb rectal ampoule. With the first method the number of samples is dependent of the number of animals in the same pen, assuring that the number of samples is representative of the group and reflect the individual variability. It must be assured that each sample correspond to one individual and it must be individually packed and stored. The fresh weight of the samples must be about 5 g, and may be stored in individual small plastic bags or any other packing system that preserves sample moisture. The samples must be stored in a refrigerator (4±1°C) until laboratory analysis.

For experimental purposes, or when possible, individual sampling may be considered, collecting faeces for all the individuals, allowing more detailed information. If the lambs are individually logged the samples may be collected from the floor, as referred previously, or from the rectal ampoule. If the lambs are logged in small groups, the faeces must be collected from the rectal amploule, which may cause a small discomfort to the animals.

### Fatty acid methyl esters analysis

In the present invention, the fatty acid composition of faeces will be analysed as their methyl esters derivatives. For that, freeze-dried faeces will react with 2 mL of sodium methoxide 0.5N solution in methanol, preferably between 10 and 30 minutes, followed by addition of 1.25M HCl in methanol and further reaction at 80°C, preferably between 15-30 minutes.

After cooling the solution, a portion of aqueous potassium carbonate will be added and fatty acid methyl esters will be extracted with an organic solvent, preferably with 2 to 4 mL of n-hexane.

Fatty acid methyl esters will be analysed by gas-chromatography with flame ionization detection using a highly polar capillary column of 100 % cyanopropylsiloxane with at least 100 meters long. The GC conditions will comprise an injector and detector temperature of 220°C and 250°C, respectively. An oven temperature program as follow: initial oven temperature of 50°C for 1 min, then increased at 50°C/min to 150°C and held 20 min, then increased at 1°C/min to 190°C, and finally increased at 2°C/min to 220°C, maintained at this temperature for 40 min.

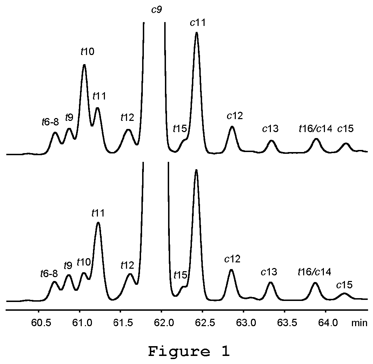

The separation of 18:1 *cis* and *trans* isomers needs to be as shown in Figure 1.

### Association between the proportion of 18:1 trans-10, 18:1 trans-11 and 18:1 trans-10/ 18:1 trans-11 ratio in faeces and rumen contents

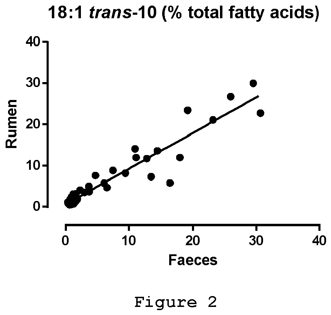

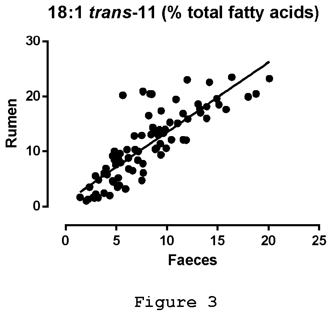

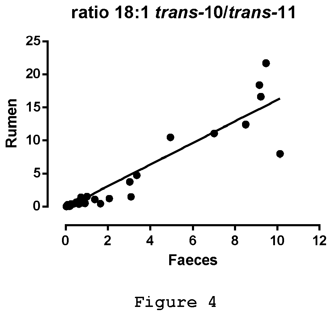

The proportions of 18:1 *trans-10,* 18:1 *trans-11* and the ratio 18:1 *trans*-10/ 18:1 *trans-11* in faeces are highly correlated to the correspondent proportions in rumen fluid, allowing the evaluation of the main drive of BH during fattening and anticipating the proportion of those BI isomers and ratio in lamb meat. These conclusions are based on the study performed with 91 lambs from 3 experiments in similar conditions. Lambs were slaughtered with 4-5 month of age and 30-45 kg live weight. Feaces were individually collected on the 24 hours before slaughter and rumen content was collected immediately after evisceration. Fatty acids from feaces and rumen content were extracted, methylated and chromatographed, to determine the proportions of 18:1 *trans-10,* 18:1 *trans*-11 and the ratio *18:1* trans-*10*/ 18:1 *trans-11.* Linear regression equations (Y=aX+b) were applied to the data to evaluate the accuracy of faeces when used as marker of the fatty acids in rumen contents. For individual fatty acids (FA), Y is the proportion of the FA in percentage of total FA in the rumen content, and X is proportion of the FA in percentage of total FA in faeces. In figures 2, 3 and 4 are represented the individual observations and the linear regressions that was better adjusted.

### Brief description of drawings

For easier understanding of this application, figures are attached in the annex that represent the preferred forms of implementation which nevertheless are not intended to limit the technique disclosed herein.
Figure 1 shows the section of a chromatogram containing the octadecenoic isomers, with clear distinction of 18:1 trans-10 and 18:ltrans-11. Fatty acids are expressed as the percentage of total fatty acids, and the ratio of 18:1 trans-10 / 18:1 trans-11 is computed.
Figure 2 shows the percentage of 18:1 trans-10 in relation to the total fatty acids and the correlation between its content in the rumen and in faeces. The respective equation is Y=0.86±0.029 X + 0.58±0.230; r²=0.9158.
Figure 3 shows the percentage of 18:1 trans-11 in relation to the total fatty acids and the correlation between its content in the rumen and in faeces. The respective equation is Y=1.27±0.093 X + 0.86±0.851; r²=0.7027
Figure 4 shows the ratio of 18:1 trans-10 / 18:1 trans-11. The respective equation is Y=1.63±0.066 X - 0.18±0.173; r2=0.8845

### Description of embodiments

Now, preferred embodiments of the present application will be described in detail, however, they are not intended to limit the scope of this application.

The present patent application describes a non-invasive method to monitoring the metabolization of polyunsaturated fatty acids (PUFA) in the rumen, which will directly affect the lipid composition of ruminant derived foods.

Thus, according to the present invention, it is disclosed herein a method for evaluating the main biohydrogenation pathways of lipids in the rumen of lambs, wherein the method comprises the following steps:
g) Collection of lamb faeces;
h) Drying the lamb faeces;
i) Preparation and extraction of the fatty acid methyl ester from the dried lamb faeces;
j) Analysis of fatty acid methyl esters by gas-chromatography with flame ionization detection equipped with a highly polar capillary column of cyanopropylsiloxane as stationary phase;
k) Identification of *cis* and *trans* octadecenoic isomers (18:1);
l) application of linear regression equations (Y=aX+b) to the 18:1 trans-10, 18:1 trans-11 proportions and the ratio 18:1 trans-10/ 18:1 trans-11 fatty acids of faeces as marker of 18:1 trans-10, 18:1 trans-11 proportions and the ratio of 18:1 trans-10/ 18:1 trans-11 fatty acids in rumen contents.
In one embodiment, the Drying step is performed by freeze-drying the lamb faeces.

According to the present invention, the ratio of 18:1 trans-10/ 18:1 trans-11 fatty acids in faeces content is used in the prediction of fatty acid composition of lamb-derived food, preferably in lamb meat.

This description is of course not in any way restricted to the forms of implementation presented herein and any person with an average knowledge of the area can provide many possibilities for modification thereof without departing from the scope defined by the claims. The following claims further define the preferred forms of implementation.

### References:

1- Aldai, N., Lavin, P., Kramer, J. K. G., Jaroso, R., & Mantecon, A. R. (2012). Breed effect on quality veal production in mountain areas: emphasis on meat fatty acid composition. Meat Science, 92, 687-696. doi: 10.1016/j.meatsci.2012.06.024
2- Bessa, R. J. B., Alves, S. P., & Santos-Silva, J. (2015). Constraints and potentials for the nutritional modulation of the fatty acid composition of ruminant meat. European Journal of Lipid Science and Technology, 117, 1325-1344. doi: 10.1002/ejlt.201400468
3- Chikwanha, O. C., Vahmani, P., Muchenje, V., Dugan, M. E. R., & Mapiye, C. (2018). Nutritional enhancement of sheep meat fatty acid profile for human health and wellbeing. Food Research International, 104, 25-38. doi: https://doi.org/10.1016/j.foodres.2017.05.005
4- Field, C. J., Blewett, H. H., Proctor, S., & Vine, D. (2009). Human health benefits of vaccenic acid. Applied Physiology Nutrition and Metabolism-Physiologie Appliquee Nutrition Et Metabolisme, 34, 979-991. doi: 10.1139/H09-079
5- Mapiye, C., Vahmani, P., Mlambo, V., Muchenje, V., Dzama, K., Hoffman, L. C., & Dugan, M. E. R. (2015). The trans-octadecenoic fatty acid profile of beef: Implications for global food and nutrition security. Food Research International, 76, 992-1000. doi: 10.1016/j.foodres.2015.05.001

## Claims

1. Method for evaluating the main biohydrogenation pathways of lipids in the rumen of lambs, comprising the following steps:
a) Collection of lamb faeces;
b) Drying the lamb faeces;
c) Preparation and extraction of the fatty acid methyl ester from the dried lamb faeces;
d) Analysis of fatty acid methyl esters by gas-chromatography with flame ionization detection equipped with a highly polar capillary column of cyanopropylsiloxane as stationary phase;
e) Identification of *cis* and *trans* octadecenoic isomers (18:1);
f) application of linear regression equation Y=aX+b to the 18:1 trans-10, 18:1 trans-11 proportions and the ratio 18:1 trans-10/ 18:1 trans-11 fatty acids of faeces as marker of 18:1 trans-10, 18:1 trans-11 proportions and the ratio of 18:1 trans-10/ 18:1 trans-11 fatty acids in rumen contents, wherein a predominance of the octadecenoic biohydrogenation isomers 18:1 *trans*-10 indicates a shift in rumen from normal 18:1 *trans*-11 biohydrogenation pathway to trans-10 shifted pathway.

2. The method for evaluating the main biohydrogenation pathways of lipids in the rumen of lambs according to the previous claim, wherein the drying step is performed by freeze-drying the lamb faeces.

3. Use of the ratio of 18:1 trans-10/ 18:1 trans-11 fatty acids in faeces content in the prediction of fatty acid composition of lamb-derived food.

## Patentansprüche

1. Verfahren zur Bewertung der wichtigsten Biohydrierungswege von Lipiden im Pansen von Lämmern, das folgende Schritte umfasst:
a) Sammlung von Lammfäkalien;
b) Trocknen der Lammfäkalien;
c) Aufbereitung und Extraktion des Fettsäuremethylesters aus getrockneten Lammfäkalien;
d) Analyse von Fettsäuremethylestern durch Gaschromatographie mit Flammenionisationsdetektion, ausgestattet mit einer hochpolaren Kapillarsäule aus Cyanopropylsiloxan als stationäre Phase;
e) Identifizierung von *cis-* und *trans*-Octadecen-Isomeren (18:1);
f) Anwendung der linearen Regressionsgleichung Y=aX+b auf die 18:1 trans-10-, 18:1 trans-11-Anteile und das Verhältnis 18:1 trans-10-/ 18:1 trans-11-Fettsäuren von Fäkalien als Marker von 18:1-trans-10-, 18:1-trans-11-Anteilen und dem Verhältnis von 18:1 trans-10-/ 18:1 trans-11-Fettsäuren im Panseninhalt, wobei ein Überwiegen der Octadecen-Biohydrierungsisomere 18:1 *trans*-10 zeigt eine Verschiebung im Pansen vom normalen 18:1 *trans*-11-Biohydrierungsweg zum *trans*-10-verschobenen Weg an.

2. Verfahren zur Bewertung der wichtigsten Biohydrierungswege von Lipiden im Pansen von Lämmern gemäß dem vorhergehenden Anspruch, wobei der Trocknungsschritt durch Gefriertrocknung der Lammfäkalien durchgeführt wird.

3. Verwendung des Verhältnisses von 18:1 trans-10-/ 18:1 trans-11-Fettsäuren im Fäkalieninhalt zur Vorhersage der Fettsäurezusammensetzung von aus Lammfleisch gewonnenen Nahrungsmitteln.

## Revendications

1. Procédé pour l'évaluation des voies de biohydrogénation principales de lipides dans le rumen d'agneaux, comprenant les étapes suivantes :
a) Collecte de fèces d'agneau ;
b) Séchage des fèces d'agneau ;
c) Préparation et extraction de l'ester méthylique d'acide gras à partir des fèces d'agneau séchées ;
d) Analyse des esters méthyliques d'acide gras par chromatographie en phase gazeuse avec détection par ionisation de flamme équipée avec une colonne capillaire hautement polaire de cyanopropyl siloxane comme phase stationnaire ;
e) Identification d'isomères octadécénoïques *cis* et *trans* (18:1) ;
f) application de l'équation de régression linéaire Y=aX+b aux proportions 18:1 trans-10, 18:1 trans-11 et au rapport 18:1 trans-10/18:1 trans-11 des acides gras des fèces comme marqueur des proportions 18:1 trans-10, 18:1 trans-11 et du rapport 18:1 trans-10/18:1 trans-11 des acides gras dans les contenus de rumen, dans lesquels une prédominance des isomères de biohydrogénation octadécénoïques 18:1 *trans*-10 indique un changement dans le rumen à partir de la voie de biohydrogénation 18:1 *trans*-11 normale jusqu'à la voie *trans*-10 changée.

2. Procédé pour l'évaluation des voies de biohydrogénation principales de lipides dans le rumen d'agneaux selon les revendications précédentes, dans lequel l'étape de séchage est exécutée par lyophilisation des fèces d'agneau.

3. Utilisation du rapport de 18:1 trans-10/18:1 trans-11 des acides gras dans le contenu des fèces dans la prédiction de la composition de l'acide gras des aliments dérivés de l'agneau.
